# EUROPÄISCHE PATENTANMELDUNG

(11) **EP 1 878 409 A2**
(43) Veröffentlichungstag der Anmeldung: **16.01.2008**
(21) Anmeldenummer: 07009235.8
(22) Anmeldetag: 08.05.2007
(51) Int. Cl.: A61F 5/37, A61G 13/00, A61B 19/00

(54) **Vorrichtung zur Ruhigstellung von unteren Extremitäten von Patienten**

(30) Priorität: 14.07.2006 DE 202006010947 U
(71) Anmelder: FORER, Karl, 39031 Pfalzen (IT)
(72) Erfinder: FORER, Karl, 39031 Pfalzen (IT)
(74) Vertreter: Samson & Partner

(57) **Zusammenfassung**

Die Erfindung betrifft eine Vorrichtung (21) zur Ruhigstellung und Stabilisierung von unter Zug gehaltener frakturierter oder lädierter unteren Extremitäten von Patienten z.B. zur Röntgen- und/oder Kernspindiagnostik, umfassend: eine längliche Auflagefläche (22) für mindestens einen Teil der unteren Extremität, mindestens eine Anlagefläche (23), die die Extremität in einer vorgegebenen anatomischen Stellung hält, wobei eine oder mehrere Anlageflächen (23) längs der Auflagefläche (22) an unterschiedlichen Stellen (51) positionierbar ist/sind.

## Beschreibung

Die Erfindung betrifft eine Vorrichtung zur Ruhigstellung und Stabilisierung von frakturierten oder lädierten unteren Extremitäten von Patienten, wie etwa Sprunggelenk, Unterschenkel, Knie, Oberschenkel samt Hüfte, zum Beispiel in der Erstversorgung (Unfallchirurgie, Erste Hilfe), zur Röntgen- oder Kernspindiagnostik, und dergleichen.

Aus den deutschen Gebrauchsmustern DE 297 03 354 U1 und DE 20 2005 012 178.9 desselben Anmelders ist eine Vorrichtung zur Immobilisierung und in Traktion gehaltener gebrochener unterer Extremitäten von Patienten bekannt. Diese dient als Stabilisierungsvorrichtung für die Röntgendiagnostik. Dabei weist die Vorrichtung eine Auflagefläche für einen Teil der frakturierten unteren Extremität auf und Anlageflächen, die z.B. den Fuß der frakturierten unteren Extremität in einer bestimmten anatomischen Winkelstellung halten.

Bekannt ist ferner, dass Frakturen der unteren Extremitäten mittels sogenannter Sandsäckchen in einer gewissen Weise ruhiggestellt werden. Bevor man die untere Extremität durch eine Gipsschiene oder mittels einer Fersenextension in die gewünschte "Frakturstellung" bringen konnte, mußte der frakturierte Teil der unteren Extremität dreibis fünfmal angehoben werden. Dies ist schmerzhaft für den Patienten und kann nachteilige Folgen, wie zum Beispiel Emboliegefahr oder Gefäßverletzungen, haben. Ferner ist dieses traditionelle Vorgehen nicht geeignet Röntgenaufnahmen anzufertigen, da die Position des z.B. frakturierten Anteils der unteren Extremität nicht ausreichend zum gesunden Teil des Beines abgrenzbar ist und zu sogenannten "schrägen" Röntgenaufnahmen führt. Ferner ist diese Ruhigstellung der Fraktur sowohl instabil als auch schmerzhaft.

Es ist eine Aufgabe der vorliegenden Erfindung eine Vorrichtung zur Ruhigstellung von unter Zug gehaltenen unteren Extremitäten in Hinblick auf eine Stabilisierung möglichst der gesamten unteren Extremität in einer anatomisch-physiologischen Stellung weiter zu verbessern.

Die Erfindung löst diese Aufgabe durch eine Vorrichtung nach dem Gegenstand von Anspruch 1. Nach einem ersten Aspekt umfaßt eine solche Vorrichtung zur Ruhigstellung von unteren Extremitäten von Patienten eine längliche Auflagefläche für mindestens einen Teil der unteren Extremität und mindestens eine Anlagefläche, die die Extremität in einer vorgegebenen anatomischen Stellung hält, wobei eine oder mehrere Anlageflächen längs und/oder quer der Auflagefläche an unterschiedlichen Stellen positionierbar ist/sind.

Weitere Aspekte und Merkmale der Erfindung ergeben sich aus den abhängigen Ansprüchen, der nachfolgenden Beschreibung von Ausführungsbeispielen und den beigefügten Zeichnungen.

Bei einem Ausführungsbeispiel sind mindestens zwei Anlageflächen am distalen Ende der Auflagefläche vorgesehen, die den angrenzenden Bereich (z.B. Fuß) der unteren Extremität in einer bestimmten anatomischen Winkelstellung halten, wobei die Stellung mindestens einer Anlagefläche bezüglich des Fußes derart veränderbar ist, dass der von den Anlageflächen gehaltene Fuß in kaudaler Richtung unter Zug gehalten ist. Hiermit erreicht man eine Vorrichtung zur Ruhigstellung und Stabilisierung von unter Zug gehaltener frakturierter oder lädierter unteren Extremitäten von Patienten z.B. zur Röntgen- und/oder Kernspindiagnostik.

Bei einem weiteren Ausführungsbeispiel ist die oder jede Anlagefläche aus einem Befestigungselement gebildet, dessen Achse senkrecht zur Auflagefläche verläuft und das exzentrisch bezüglich seiner Achse drehbar gelagert ist. Bevorzugt ist jedes Befestigungselement über einen Stift, der im wesentlichen senkrecht aus der Auflagefläche herausragt, mit der Auflagefläche verbunden.

Bei einem weiteren Ausführungsbeispiel weist die Auflagefläche im Randbereich der beiden Längsseiten eine Vielzahl von gegenüberliegenden Bohrungen auf, die für die Aufnahme von Befestigungselementen vorgesehen sind.

Bei einem weiteren Ausführungsbeispiel hat jedes Befestigungselement zumindest eine Bohrung, wobei diese Bohrungen parallel zur Kegelachse sind, die Bohrungen exzentrisch zu der Achse des jeweiligen Befestigungselementes ist und diese Bohrungen jeweils einen Stift aufnehmen, der senkrecht aus der Auflagefläche herausragt.

Bei einem weiteren Ausführungsbeispiel ist die Auflagefläche an einer oder mehreren Stellen abgewinkelt, derart, dass die untere und/oder obere Extremität des Patienten in einer anatomisch physiologischen Stellung aufliegen kann. Alternativ verläuft die Auflagefläche am proximalen Ende in einem Bogen zur Stellfläche hin. Hiermit erreicht man eine optimale Lagerung auch der proximalen Abschnitte der unteren Extremität wie z.B. Oberschenkel samt Hüfte.

Bei einem weiteren Ausführungsbeispiel verläuft die Auflagefläche vom distalen Ende in einem Winkel im Bereich von etwa von etwa 2° bis 10° ansteigend bis zu etwa ¾ der Länge und fällt dann zum proximalen Ende hin leicht ab.

Bei einem weiteren Ausführungsbeispiel weist die Auflagefläche eine Stützeinrichtung am distalen Ende auf, welche die Auflagefläche in einem Abstand zur Untergrund hält. Auf diese Weise kann eine Verlängerung einer Patientenliegefläche simuliert werden

Bei einem weiteren Ausführungsbeispiel ist die Auflagefläche am proximalen Ende derart abgewinkelt, dass sie sich im wesentlichen vertikal zum Untergrund erstreckt.

Bei einem weiteren Ausführungsbeispiel ist die Stellung zweier gegenüberliegender Anlageflächen bezüglich des zugehörigen Abschnittes der unteren Extremität derart veränderbar, dass die Anlageflächen die Extremität unter Zug in distaler Richtung bezüglich der Achse der Fraktur halten. Bevorzugt halten die beiden Anlageflächen zwischen sich den jeweiligen Abschnitt der Extremität und stehen unter Spannung zueinander.

Bei einem weiteren Ausführungsbeispiel ist die Vorrichtung vollständig aus Polymethylmetacrylat gefertigt ist.

In einem weiteren Ausführungsbeispiel sind die Befestigungselemente Kegelelemente und weisen Bohrungen auf, wobei diese Bohrungen parallel zur Kegelachse sind, die Bohrungen exzentrisch zu der Achse des jeweiligen Kegelelementes ist und diese Bohrungen jeweils einen Stift aufnehmen der senkrecht aus der Auflagefläche herausragt. Um die Vorrichtung auch in der Kernspintomografie bzw. Magnetresonanztomografie einsetzbar zu machen, sind die Stifte aus einem magnetresonanztauglichen Material, wie z.B. Fiberglas, gefertigt.

Ferner ist an der erfindungsgemäßen Vorrichtung im Einsatz für die Kernspintomografie besonders vorteilhaft, dass die beschriebene Ruhigstellung den z.B. lädierten oder frakturierten Anteil der unteren Extremität sehr schnell und eindeutig blockiert und gleichzeitig fixiert, ohne dass hierbei z.B. eine Gipsschiene erforderlich wäre.

Die erfindungsgemäße Vorrichtung eignet sich aber auch für die Ruhigstellung von nicht-frakturierten (z.B. Bandverletzungen) unteren Extremitäten z.B. bei einer Kernspintomographie oder Röntgenaufnahmen.

Ferner ist die erfindungsgemäße Vorrichtung so konzipiert, dass man mit ihr die Kernspin- und Röntgendiagnostik durchführen kann, ohne dass die Magnetfeld- bzw. Röntgendosis für den Patienten erhöht zu werden braucht.

Weitere Aspekte und Merkmale der Erfindung ergeben sich aus den abhängigen Ansprüchen, der nachfolgenden Beschreibung von Ausführungsbeispielen und den beigefügten Zeichnungen.

### KURZBESCHREIBUNG DER ZEICHNUNG

Ausführungsformen der Erfindung werden nun beispielhaft und unter Bezugnahme auf die angefügten Zeichnungen beschrieben, in denen zeigt schematisch:
Fig. 1 eine perspektivische Ansicht eines Ausführungsbeispiels einer erfindungsgemäßen Vorrichtung;
Fig. 2 eine Schnittansicht des Ausführungsbeispiels von Fig. 1 entlang der Linie A;
Fig. 3 eine Detailansicht einer Auflagefläche der erfindungsgemäßen Vorrichtung; und
Fig. 4 eine frontale Teilansicht der Auflagefläche.

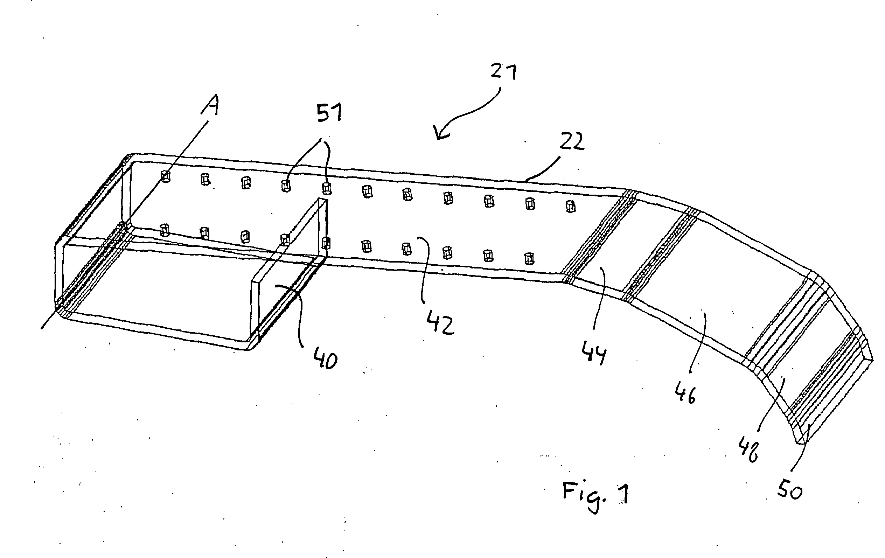

In den Fig. 1 bis 4 weist das Ausführungsbeispiel der erfindungsgemäßen Vorrichtung 21 eine Auflagefläche 22 und Anlageflächen 23 auf, die wie nachfolgend beschrieben, an mehreren vorbestimmten Stellen der Auflagefläche 22 montierbar und wieder entfernbar sind.

Die Auflagefläche 22 dient als Auflage einer z.B. gebrochenen unteren Extremität unabhängig davon, ob es sich um das rechte oder linke Bein, ob es sich um Erwachsene oder Patienten im Kindesalter handelt, etc. so dass sich die Fraktur immer in einer mittleren Stellung auf der Auflagefläche 22 befindet.

In Fig. 1 ist die Auflagefläche 22 der erfindungsgemäßen Vorrichtung 21 (ohne montierte Anlageflächen 23) näher dargestellt. Die Auflagefläche 22 wird an ihrem distalen (körperfernen) Ende von einer kastenförmigen Stützeinrichtung 40 getragen, auf welcher sich die Auflagefläche 22 mit ihrem distalen Ende abstützt, so dass die Auflagefläche 22 in einem Abstand zu einer Stellfläche gehalten wird. Die Auflagefläche 22 umfaßt einen ersten Abschnitt 42, der sich in einem leicht ansteigenden Winkel von etwa 5° vom distalen Ende aus zur proximalen Seite (körpernahe Seite) hin erstreckt und an welchem sich nacheinander ein zweiter Abschnitt 44, ein dritter Abschnitt 46, ein vierter Abschnitt 48 und ein fünfter Abschnitt 50 anschließen. Diese Abschnitte sind jeweils leicht gewinkelt zueinander angeordnet und bewirken einen insgesamt gekrümmten Verlauf der Auflagefläche 22 von dem proximalen Ende des ersten Abschnittes 42 bis zum freien Ende des fünften Abschnittes 50, auf welchem sich die Auflagefläche 22 auf einer Stellfläche abstützt. Dieser gekrümmte bzw. abgewinkelte Verlauf der Auflagefläche 22 an deren proximalen Ende hat den Vorteil, dass sich diese der anatomisch-physiologischen Stellung der gelagerten unteren Extremität eines Patienten, insbesondere einer abgewinkelten Beinstellung, anpasst und den Oberschenkelbereich des Patienten mit abstützt. Diese zusätzliche Abstützung verbessert auch die Fixierung der frakturierten unteren Extremität und wirkt damit schmerzlindernd.

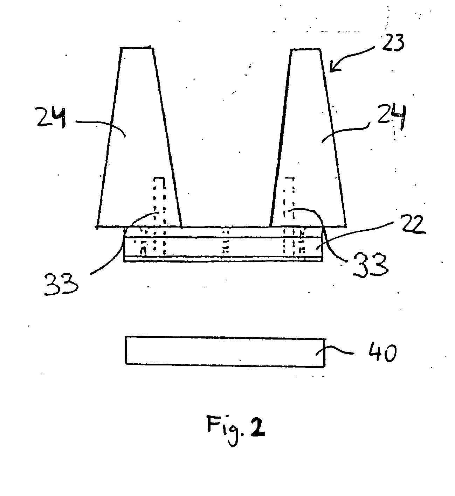

Der erste Abschnitt 42 der Auflagefläche 22 weist in einem Abstand zu den beiden Längsseiten eine Vielzahl von Bohrungen 51 auf, die für die Aufnahme der Anlageflächen 23 (siehe Fig. 2) vorgesehen sind. Im vorliegenden Ausführungsbeispiel werden die Anlageflächen 23 von den Kegelflächen zweier gegenüberliegender Kegelelementen 24 gebildet, die in zwei gegenüberliegenden der vorgenannten Bohrungen 51 einsetzbar sind. Je nach Bedarf können mehrere dieser Kegelpaare 24 montiert werden, um je nach Wunsch und medizinischer Notwendigkeit den entsprechenden Abschnitt der gelagerten Extremität des Patienten stabil zu fixieren. Bei Verwendung mehrere Kegelpaare entlang der Auflagefläche 22 ist es möglich, die gesamte frakturierte Extremität, jedenfalls entlang der anatomischen Achse der Fraktur, zu stabilisieren und gegebenenfalls unter Zug zu bringen. Die untere Extremität wird stabilisiert und in der gewünschten Position gehalten. Dadurch ist der Patient mit den geringsten Schmerzen für eine Röntgendiagnostik bzw. für eine nachfolgende Operation optimal gelagert.

In Fig. 2 sind die vorgenannten Kegelelemente 24 im Detail dargestellt. Die Anlageflächen 23 umfassen zwei Kegelelemente 24, von denen jedes einzelne drehbar exzentrisch zur Kegelachse gelagert ist. Die zwei Kegelelemente 24 oder weitere Kegelelemente 24 können in jede beliebige der Bohrungen 51 der Auflagefläche 22 (siehe Fig. 1) eingesetzt werden. Natürlich werden die Kegelpaare 24 in jeweils gegenüberliegender Lage in die Bohrungen 51 der Auflagefläche 22 eingesetzt. Mit einer jeweils parallel zur Kegelelementachse hineinragenden Bohrung 51 werden die Kegelelemente 24 mittels Stiften 32, 33, die senkrecht aus der Auflagefläche 22 herausragen, derart auf der Auflagefläche 22 gelagert, dass die Kegelelemente 24 um eine Achse drehbar sind, die senkrecht zur Auflagefläche 22 ist und exzentrisch zur Achse der Kegelelemente 24 ist.

Die Stifte 32, 33 sind aus einem nicht-magnetischen Material gefertigt, so dass sie z.B. Kernspintomografieaufnahmen nicht beeinflussen. Ein hierzu geeignetes Material ist beispielsweise Fiberglas.

Die Drehachsen 26 der Kegelelemente 24 fallen jeweils mit den Achsen der Stifte 32 und 33 zusammen. Legt man den Fuß eines Patienten zwischen die beiden distalen Anlageflächen 23, die von den zwei Kegelelementen 24 am Fußende der Auflagefläche 22 gebildet werden, und dreht man die beiden Kegelelemente 24 derart, dass der Fuß unter Zug gehalten wird (in kaudaler Richtung) und zwar bezüglich der Achse der z.B. frakturierten unteren Extremität, wird der Fuß in einer garantiert anatomischen Winkelstellung stabil gelagert.

Als Folge der gegenseitigen Annäherung der Kegelelemente 24 verbleibt z.B. der Fuß zwischen den Kegelelementen 24 in Ruhestellung. Um beide Kegelelemente 24 in einer blokkierenden Position zu halten, das heißt in der den Fuß am nächsten liegenden Position (sei es Metatarsale 1 und Metatarsale 5), werden die Kegelelemente 24 mittels geeigneter Mittel, wie zum Beispiel einem Gummiband, an ihrer Rückwärtsbewegung, das heißt einer gegenseitig auseinanderdriftenden Bewegung, gehindert.

Eine ähnlich fixierende und stabilisierende Wirkung erzielt man mit Kegelelementen 24, die zusätzlich in anderen weiter proximal liegenden Positionen entlang der Auflagefläche 42 in den dort vorgesehenen Bohrungen 51 angeordnet und in entsprechender Weise in einer blockierenden Position in Bezug auf die untere Extremität gehalten sind. Dieser Vorgang bewirkt eine verstärkte Traktion entlang der unteren Extremität, insbesondere entlang der anatomischen Achse einer Fraktur, was eine Schmerzlinderung für den Patienten herbeiführt.

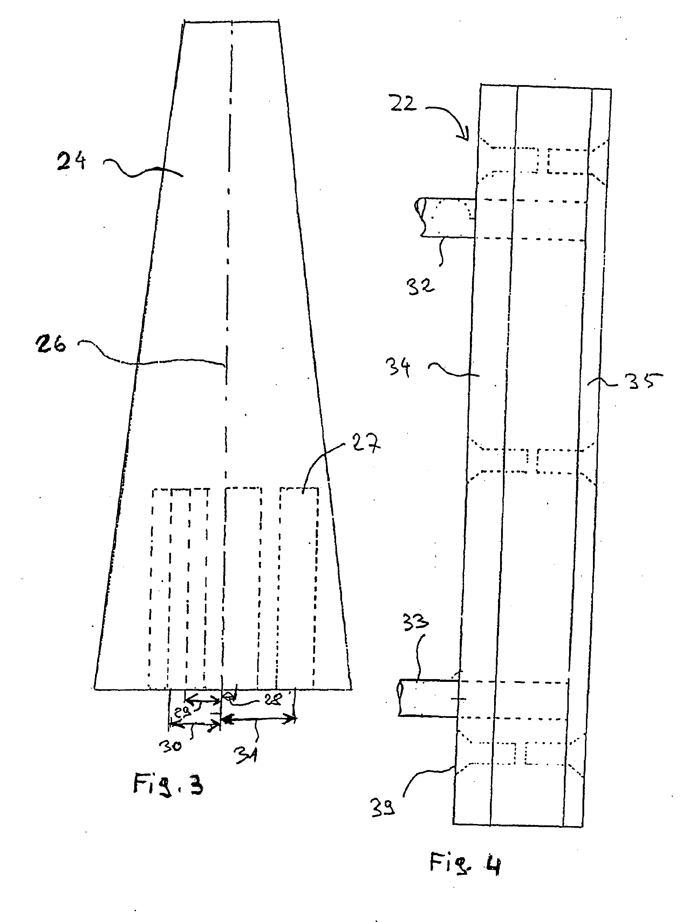

Gemäß Fig. 3 weist die Mantelfläche der Kegelelemente zur Auflagefläche 22 einen Winkel α im Bereich zwischen 95° und 115° auf, wobei der Winkel vorzugsweise 97° ist. Die zur Achse der Kegelelemente 24 parallel hineinragenden Bohrungen sind von der Kegelelementachse 26 in einem Bereich zwischen 5 mm und 20 mm entfernt, wobei in diesem Bereich auch mehrere Bohrungen vorgesehen sein können. Der Abstand der aus der Auflagefläche 22 herausragenden Stifte ist in einem Bereich zwischen 110 mm und 140 mm, wobei er vorzugsweise 120 mm ist.

Die Auflagefläche 22 ist, bis auf etwaige röntgendichte Markierungen für Maßeinheiten, aus einem für Röntgenstrahlung durchlässigen Material hergestellt. Ferner können auch die weiteren Bestandteile der Vorrichtung aus einem für die Röntgenstrahlung durchlässigen Material hergestellt sein. Beispielsweise kann die Vorrichtung aus PMMA (Polymetylacrylat) hergestellt sein.

Gemäß Fig. 4 kann die Auflagefläche 22 eine obere 34 und eine untere 35 Platte aufweisen, die auf den kurzen Seiten beabstandet sind. Zu diesem Zweck ist am proximalen Teil der Auflagefläche 22 zwischen den beiden Platten 34 und 35 ein Zwischenstück 36 eingefügt, an dem verschraubt oder verklebt die Platten 34 und 35 anliegen. Am distalen Ende der Auflagefläche 22 zwischen den Platten 34 und 35 ist ein Abstandshalter 38 eingefügt, der die gleichen Abmessungen wie der Abstandshalter 36 hat, wobei die jeweiligen Platten mit diesem Abstandshalter wiederum verschraubt oder verklebt verbunden sind.

Als mögliche Abmessungen können die beiden Stifte 32 und 33, auf denen die Kegelelemente 24 exzentrisch gedreht aufliegen, einen Durchmesser von 10 mm und eine Gesamtlänge von 80 mm aufweisen. Diese Stifte werden um 30 mm in die Auflagefläche 22 eingelassen, so dass sie 50 mm aus dieser Fläche senkrecht herausragen. Die Bohrungen der Kegelelemente 24 besitzen einen Durchmesser von 10 mm und eine Länge von 50 mm, so dass sie die Stifte 32 bzw. 33 aufnehmen können. Verschiedene exzentrische Bohrungen 27 können beispielsweise 5 mm, 10 mm, 15 mm und 20 mm vom Achsenzentrum des Kegels entfernt angebracht sein.

Gemäß einem weiteren Ausführungsbeispiel sind die Stifte 32 und 33 in dem aus der Auflagefläche 22 herausragenden Abschnitt mit einem größeren Durchmesser gefertigt, als der Durchmesser desjenigen Abschnittes, der in die Auflagefläche 22 hineinragt (siehe strichpunktierte Linien in Fig. 6). Auf diese Weise steht der herausragende Abschnitt der Stifte 32 und 33 geringfügig über die Umrandung der entsprechenden Bohrung in der Auflagefläche 22 hinaus, so dass die Bohrung und damit das Innere der Auflagefläche 22 durch den Bolzen selbst und das Material der Auflagefläche dichtend verschlossen ist. Ein etwaiger Flüssigkeitseintritt, wie z.B. Blut, Desinfektionsmittel etc., wird einfach und effizient ohne zusätzliche Dichtmittel verhindert.

## Patentansprüche

1. Vorrichtung zur Ruhigstellung von unteren Extremitäten von Patienten, umfassend:
eine längliche Auflagefläche (22) für mindestens einen Teil der unteren Extremität,
mindestens eine Anlagefläche (23), die die Extremität in einer vorgegebenen anatomischen Stellung hält,
**dadurch gekennzeichnet, dass**
eine oder mehrere Anlageflächen (23) längs der Auflagefläche (22) an unterschiedlichen Stellen (51) positionierbar ist/sind.

2. Vorrichtung nach Anspruch 1, bei welcher mindestens zwei Anlageflächen (23) am distalen Ende der Auflagefläche (22) vorgesehen sind, die den angrenzenden Bereich der unteren Extremität in einer bestimmten anatomischen Winkelstellung halten, wobei die Stellung mindestens einer Anlagefläche (23) bezüglich des Fußes derart veränderbar ist, dass der von den Anlageflächen (23) gehaltene Fuß in kaudaler Richtung unter Zug gehalten ist.

3. Vorrichtung nach Anspruch 1 oder 2, bei welcher die oder jede Anlagefläche (23) aus einem Befestigungselement (24) gebildet wird, dessen Achse senkrecht zur Auflagefläche (22) verläuft und das exzentrisch bezüglich seiner Achse drehbar gelagert ist.

4. Vorrichtung nach Anspruch 3, bei welcher jedes Befestigungselement (24) über einen Stift (32,33), der im wesentlichen senkrecht aus der Auflagefläche (22) herausragt, mit der Auflagefläche (22) verbunden ist.

5. Vorrichtung nach Anspruch 4, bei welcher die Auflagefläche (22) im Randbereich der beiden Längsseiten eine Vielzahl von gegenüberliegenden Bohrungen (51) aufweist, die für die Aufnahme von Befestigungselementen (24) vorgesehen sind.

6. Vorrichtung nach Anspruch 3, wobei jedes Befestigungselement (24) zumindest eine Bohrung (27) aufweist, diese Bohrungen (27) parallel zur Kegelachse sind (26), die Bohrungen (27) exzentrisch zu der Achse des jeweiligen Befestigungselementes (24) ist und diese Bohrungen jeweils einen Stift (32,33) aufnehmen, der senkrecht aus der Auflagefläche (22) herausragt.

7. Vorrichtung nach einem der Ansprüche 4 bis 6, wobei die Stifte (32, 33) aus einem antimagnetischen Material hergestellt sind.

8. Vorrichtung nach Anspruch 7, wobei die Stifte (32, 33) aus Fiberglas oder Nylon hergestellt sind.

9. Vorrichtung nach einem der vorstehenden Ansprüche, bei welcher die Auflagefläche (22) an einer oder mehreren Stellen abgewinkelt ist, derart, dass die untere und/oder obere Extremität in einer anatomisch physiologischen Stellung aufliegen kann.

10. Vorrichtung nach einem der vorstehenden Ansprüche, bei welcher die Auflagefläche (22) am proximalen Ende in einem Bogen zur Stellfläche hin verläuft.

11. Vorrichtung nach Anspruch 9 oder 10, bei welcher sich die Auflagefläche (22) vom distalen Ende in einem Winkel im Bereich von etwa von etwa 2° bis 10° ansteigend bis zu etwa ¾ der Länge erstreckt und dann zum proximalen Ende hin leicht abfällt.

12. Vorrichtung nach einem der vorstehenden Ansprüche, bei welcher die Auflagefläche (22) eine Stützeinrichtung (40) am distalen Ende aufweist, welche die Auflagefläche (22) in einem Abstand zur Untergrund hält.

13. Vorrichtung nach einem der Ansprüche 9 bis 12, bei welcher die Auflagefläche (22) am proximalen Ende derart abgewinkelt ist, dass sie sich im wesentlichen vertikal zum Untergrund erstreckt.

14. Vorrichtung nach einem der vorstehenden Ansprüche, bei welcher die Stellung zweier gegenüberliegende Anlageflächen (23) bezüglich des zugehörigen Abschnittes der unteren Extremität derart veränderbar ist, dass die Anlageflächen (23) die Extremität unter Zug in distaler Richtung bezüglich der Achse der Fraktur halten.

15. Vorrichtung nach Anspruch 14, bei welcher die beiden Anlageflächen (23) zwischen sich den jeweiligen Abschnitt der Extremität halten und unter Spannung zueinander stehen.

16. Vorrichtung nach einem der vorstehenden Ansprüche, wobei die Vorrichtung aus Polymethylmetacrylat gefertigt ist.
